(19) Europäisches Patentamt
European Patent Office
Office européen des brevets

(11) EP 3 478 666 B1

(12) EUROPEAN PATENT SPECIFICATION

(45) Date of publication and mention of the grant of the patent:
**29.07.2020 Bulletin 2020/31**

(21) Application number: **17740823.4**

(22) Date of filing: **26.06.2017**

(51) Int Cl.:
***C07D 233/58*** (2006.01) ***C08F 220/10*** (2006.01)
***C09D 133/06*** (2006.01) ***C09D 4/06*** (2006.01)

(86) International application number:
**PCT/US2017/039181**

(87) International publication number:
**WO 2018/005311 (04.01.2018 Gazette 2018/01)**

# (54) POLYMERIZABLE IONIC LIQUID COMPOSITIONS

POLYMERISIERBARE IONISCHE FLÜSSIGKEITSZUSAMMENSETZUNGEN

COMPOSITIONS LIQUIDES IONIQUES POLYMÉRISABLES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.06.2016 US 201662356011 P**

(43) Date of publication of application:
**08.05.2019 Bulletin 2019/19**

(73) Proprietor: **3M Innovative Properties Company**
**St. Paul, MN 55133-3427 (US)**

(72) Inventors:
- **SHAFER, Kathleen S.**
  **Saint Paul**
  **Minnesota 55133-3427 (US)**
- **LEWANDOWSKI, Kevin M.**
  **Saint Paul**
  **Minnesota 55133-3427 (US)**
- **STRAND, John T.**
  **Saint Paul**
  **Minnesota 55133-3427 (US)**
- **VAN LENGERICH, Henrik B.**
  **Saint Paul**
  **Minnesota 55133-3427 (US)**

(74) Representative: **Mathys & Squire**
**Mathys & Squire Europe LLP**
**Theatinerstraße 7**
**80333 München (DE)**

(56) References cited:
**US-A1- 2009 029 162**
**US-A1- 2011 288 227**
**US-A1- 2012 101 184**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 478 666 B1

## Description

### Field of the Invention

The present disclosure is directed to water removable coating compositions according to claims 1 to 7 or claims 9 to 12, to a coated article according to claim 8 and to methods of replicating a mold according to claims 13 to 15.

### Background

Ionic liquids (ILs) are salts in which the cation and anion are poorly coordinated. At least one of the ionic components is organic and one of the ions has a delocalized charge. This prevents the formation of a stable crystal lattice, and results in such materials existing as liquids, often at room temperature, and at least, by definition, at less than 100°C. For example, sodium chloride, a typical ionic salt, has a melting point of about 800°C, whereas the ionic liquid N-methylimidazolium chloride has a melting point of about 75°C.

Ionic liquids typically comprise an organic cation, such as a substituted ammonium or a nitrogen-containing heterocycle, such as a substituted imidazolium, coupled with an inorganic anion. However, species have also been described wherein the cation and anion are organic. When the ionic liquid comprises at least one polymerizable group, it is a polymerizable ionic liquid ("PIL").

Some of the features of ionic liquids that have caused the considerable research and development efforts in recent years include a broad liquid range and their high dissolving power - ionic liquids are excellent solvents for both organic and inorganic materials. Additionally, ionic liquids have high polarity, high thermal stability, high refractive indices, and high ionic conductivity. They are also non-flammable and have negligible vapor pressure. As a reaction medium, ionic liquids have been shown to accelerate many organic reactions including Diels-Alder cycloaddition reactions and alkylation reactions.

The combination of polymerizable functionality, room temperature organic liquid salt characteristics, high refractive index, high ionic conductivity, and negligible vapor pressure may enable their incorporation into new high value functional materials. Because of their low volatility, non-flammability, high solvency attributes, and potential ability to be recycled, ionic liquids have been promoted as environmentally safe or "green" solvents to replace conventional organic solvents (volatile organic compounds, VOCs).

US 2011/288227 A1, US 2009/029162 A1 and US 2012/101184 A1 relate to further different ionic liquid for coating compositions.

### Summary

The present disclosure provides water removable coating compositions which are based on polymerizable ionic liquid compounds of the cation of the formula, or conjugate base thereof:

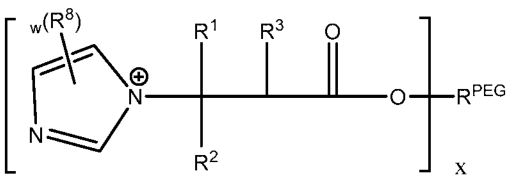

I

$R^1$ is H or a $C_1$-$C_{25}$ alkyl group,
$R^2$ is H or -CO- $X^1$-$R^5$, where $R^5$ is a H, a $C_1$-$C_{25}$ alkyl group or $R^{PEG}$ and $X^1$ is -O- or - $NR^6$-, where $R^6$ is H or a $C_1$-$C_6$ alkyl;
$R^3$ is H or $CH_3$,
$R^8$ is a (hetero)hydrocarbyl group, and w is 0-3, preferably 0; and
$R^{PEG}$ is a poly(alkyleneoxy) containing group; and
subscript x is 1 or 2.

In some embodiments, a mixture of polymerizable ionic liquid compounds where subscript x is 1 and 2 is desirable. Such mixture can increase the ionic crosslink content of coatings derived therefrom improving the water removability while retaining the swellability.

The present disclosure provides polymerizable ionic liquid compositions that are useful in a variety of adhesive and coating applications. The compositions comprise an acid functional monomer or acid-functional copolymer (or

conjugate base thereof), and an imidazole compound of formula I (or conjugate base thereof). The composition is formed by acid-base interactions between the acid groups of the monomer and the imidazole compound, and by free radical polymerization of the ionic liquid monomer. The crosslinked compositions are exceptionally hydrophilic, and easily removed from a substrate by contact with water.

In one embodiment, the composition is a polymerizable ionic liquid comprising a polymerizable anion including an acid-functional monomer and a cation corresponding to the conjugate acid of the imidazole compound of Formula I.

In another embodiment this disclosure provides a curable composition comprising an acid-functional monomer and a compound of Formula I. In one embodiment the curable composition may comprise the imidazole compound of formula I, and acid functional monomer, and optionally other monomers as described herein. Such composition may be polymerized using free radical initiators. In another embodiment the curable compositions may comprise a syrup polymer composition comprising a solute acid functional copolymer, a solvent acid functional monomer and the imidazole compounds of Formula I, which form an acid-base interaction between the imidazole group(s) and the acid groups of the acid functional copolymer.

In another embodiment a coating composition is provided comprising an acid-functional copolymer and the imidazole compounds of Formula I.

The present disclosure provides curable acrylic resin compositions that can be coated using cast and cure techniques to yield nano- or microreplicated acrylic films on a substrate. These microreplicated films can be etched and metalized to give a patterned film, and finally the non-metalized excess acrylic film and overcoated metal can be subsequently removed from the substrate using only a water wash, leaving a nano- or microscale patterned film.

Coatings or films derived from the crosslinkable compositions are capable of being swelled by the application of solvent, conveniently water. These compositions are readily cast and cured on convenient substrates, and yet the polymerized film will swell and lift off or detach from those substrates when desired by means of a simple water wash or contact. By "swell" or "swellable", the coating compositions will absorb at least 100 wt.% water on contact. In some embodiments, the compositions will absorb at least 200 wt.% or 300 wt.% water. Swelling is very rapid, occurring within 30 second of contact with water. In some embodiments, the swelled coating will break up into small pieces upon contact.

The present disclosure further provides methods for molding using these compositions. In another embodiment, the present disclosure provides a method of forming a predetermined metalized pattern on a substrate, comprising: casting and curing the composition into a first side of the substrate so as to form a water removable layer on the substrate, the water removable layer having thick portions and thin portions such that the thin portions correspond to the predetermined pattern; optionally etching the water removable layer so that the thin portions are removed and substrate is partially exposed; metalizing the first side so as to deposit metal onto the exposed portions of the substrate and onto the thick portions of the water removable layer; and exposing the water removable layer to water, releasing the water removable layer from the substrate and leaving the metalized pattern.

As used herein:

"acryloyl" is used in a generic sense and mean not only derivatives of acrylic acid, but also amine, and alcohol derivatives, respectively;

"(meth)acryloyl" includes both acryloyl and methacryloyl groups; i.e. is inclusive of both esters and amides.

"poly(meth)acryloyl" means a compound having two or more (meth)acryloyl groups that may function as Michael acceptors.

"curable" means that a coatable material can be transformed into a solid, substantially non-flowing material by means of cooling (to solidity hot melts), heating (to dry and solidify materials in a solvent), chemical cross linking, radiation crosslinking, or the like.

"alkyl" includes straight-chained, branched, and cyclic alkyl groups and includes both unsubstituted and substituted alkyl groups. Unless otherwise indicated, the alkyl groups typically contain from 1 to 20 carbon atoms. Examples of "alkyl" as used herein include, but are not limited to, methyl, ethyl, n-propyl, n-butyl, n-pentyl, isobutyl, t-butyl, isopropyl, n-octyl, n-heptyl, ethylhexyl, cyclopentyl, cyclohexyl, cycloheptyl, adamantyl, and norbornyl, and the like. Unless otherwise noted, alkyl groups may be mono- or polyvalent.

"heteroalkyl" includes both straight-chained, branched, and cyclic alkyl groups with one or more heteroatoms independently selected from S, O, and N with both unsubstituted and substituted alkyl groups. Unless otherwise indicated, the heteroalkyl groups typically contain from 1 to 20 carbon atoms. "Heteroalkyl" is a subset of "hydrocarbyl containing one or more S, N, O, P, or Si atoms" described below. Examples of "heteroalkyl" as used herein include, but are not limited to, methoxy, ethoxy, propoxy, 3,6-dioxaheptyl, 3-(trimethylsilyl)-propyl, 4-dimethylaminobutyl, and the like. Unless otherwise noted, heteroalkyl groups may be mono- or polyvalent.

"aryl" is an aromatic group containing 6-18 ring atoms and can contain optional fused rings, which may be saturated, unsaturated, or aromatic. Examples of an aryl groups include phenyl, naphthyl, biphenyl, phenanthryl, and anthracyl. Heteroaryl is aryl containing 1-3 heteroatoms such as nitrogen, oxygen, or sulfur and can contain fused rings. Some examples of heteroaryl groups are pyridyl, furanyl, pyrrolyl, thienyl, thiazolyl, oxazolyl, imidazolyl, indolyl, benzofuranyl, and benzthiazolyl. Unless otherwise noted, aryl and heteroaryl groups may be mono- or polyvalent. "(hetero)hydrocarbyl" is inclusive of hydrocarbyl alkyl and aryl groups, and heterohydrocarbyl heteroalkyl and heteroaryl groups, the later comprising one or more catenary oxygen heteroatoms such as ether or amino groups. Heterohydrocarbyl may optionally contain one or more catenary (in-chain) functional groups including ester, amide, urea, urethane, and carbonate functional groups. Unless otherwise indicated, the non-polymeric (hetero)hydrocarbyl groups typically contain from 1 to 60 carbon atoms. Some examples of such heterohydrocarbyls as used herein include, but are not limited to, methoxy, ethoxy, propoxy, 4-diphenylaminobutyl, 2-(2'-phenoxyethoxy)ethyl, 3,6-dioxaheptyl, 3,6-dioxahexyl-6-phenyl, in addition to those described for "alkyl", "heteroalkyl", "aryl", and "heteroaryl" *supra*.

## Brief Description Of The Figures

The disclosure may be more completely understood in consideration of the following detailed description of various embodiments of the disclosure in connection with the accompanying figures, in which:

**FIG. 1** is a side view of an exemplary substrate ready to receive a patterned coating of a water removable composition.

**FIG. 2** is a side view of the substrate of **FIG. 1** with the patterned coating of a water removable composition disposed on one side.

**FIG. 3** is a side view of the substrate of **FIG. 2** after an etching process has been performed to remove the thin portions of the patterned coating.

**FIG. 4** is a side view of the substrate of **FIG. 3** after a metallizing process has been performed.

**FIG. 5** is a side view of the substrate of **FIG. 4** after a water wash has been used to remove the remaining portions of the patterned coating.

## Detailed Description

The imidazole compounds of Formula I are Michael addition products of an imidazole compound and a Michael acceptor compound; i.e. a (meth)acrylate ester of a poly(alkylene oxides, such as a polyethylene glycol (meth)acrylate. Such compounds may be prepared as described in Scheme I.

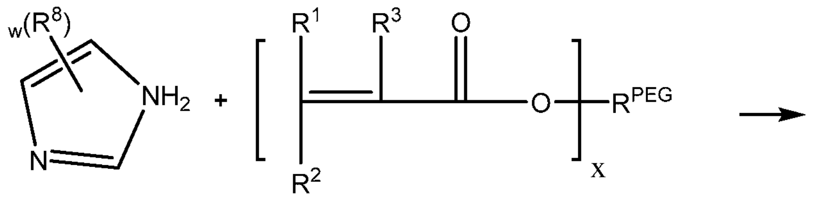

I

where

$R^1$ is H or a $C_1$-$C_{25}$ alkyl group,
$R^2$ is H or -CO- $X^1$-$R^5$, where $R^5$ is a H, a $C_1$-$C_{25}$ alkyl group or $R^{PEG}$ and $X^1$ is -O- or - $NR^6$-, where $R^6$ is H or a $C_1$-$C_6$ alkyl;
$R^3$ is H or $CH_3$,
$R^8$ is a (hetero)hydrocarbyl group, and w is 0, 1, 2 or 3; and
$R^{PEG}$ is a poly(alkyleneoxy) containing group; and
subscript x is 1 or 2.

Generally the molar ratio of the imidazole cation to the anionic acid groups of the acid-functional monomer or copolymer is approximately equimolar +/- 20%.
As can be seen, the compounds of Formula I in Scheme I may have one or two imidazole groups, enabling crosslinking of acid-functional copolymers by acid-base interaction. Compositions including compounds of Formula I may be combined with other monomer or acid-functional copolymers, as will be described further.
In some embodiments, the $R^{PEG}$ group may include an ethoxylated or propxylated bisphenol A or bisphenol C

groups.

As illustrated *supra,* the compounds of Formula I may be prepared by Michael addition of an imidazole compound to a poly(alkylene oxide) mono- or di(meth)acrylate. Useful polyacryloyl compounds include those of the general formula:

$$R^{PEG}\text{-}(X^1\text{-}C(O)\text{-}CR^3{=}CR^1R^2)_y \qquad \text{II}$$

wherein each $X^1$ is selected from alkylene, -O-, or -$NR^6$- where each $R^6$ independently represents H or an alkyl group having from 1 to 6 carbon atoms;
$R^{PEG}$ is a poly(alkyleneoxy) containing group, preferably containing poly(ethylene oxide) or poly(propylene oxide) units, or mixtures thereof.
subscript y is one or two.

The "PEG" or poly(alkylene oxide) containing group of Formulas I-II are generally derived from compounds of the formula:

$$HO\text{-}\ CH(R^4)\text{-}CH_2\text{-}O\text{-}(CH(R^4)\text{-}CH_2\text{-}O)_m\text{-}CH(R^4)\text{-}CH_2\text{-}O\text{-}R^{10}, \qquad \text{III}$$

wherein $R^4$ is a H or a $C_1$ to $C_4$ alkyl group,
m may be zero, preferably from 1 to 500, more preferably 2 to 100, most preferably 2 to 30, and
$R^{10}$ is H or a $C_1$-$C_4$ alkyl.

With respect to the useful polyacryl compounds of Formula II, it will be understood that the corresponding amides or thioesters are also useful. The multifunctional ethylenically unsaturated monomer is preferably a PEG ester of acrylic acid.

Preferred multifunctional ethylenically unsaturated esters of acrylic acid and can be described by the formula:

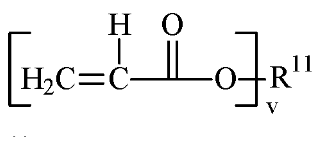
IV

$R^{11}$ is poly(alkylene oxide) such as derived from the compound of Formula III.
v is 1 or 2.

The anionic monomers of the polymerizable ionic liquid have an ethylenically unsaturated polymerizable groups and an acid group. The acid functional group may be an acid *per se,* such as a carboxylic acid, or a portion may be the conjugate base thereof. In the presence of the imidazole compound, these acid functional monomers form the conjugate base.

Useful acid functional monomers include, but are not limited to, those selected from ethylenically unsaturated carboxylic acids, ethylenically unsaturated sulfonic acids, ethylenically unsaturated phosphonic acids, and mixtures thereof. Examples of such compounds include those selected from acrylic acid, methacrylic acid, itaconic acid, fumaric acid, crotonic acid, citraconic acid, maleic acid, oleic acid, β-carboxyethyl (meth)acrylate, 2-sulfoethyl methacrylate, styrene sulfonic acid, 2-acrylamido-2-methylpropanesulfonic acid, vinylphosphonic acid, and mixtures thereof.

Due to their availability, acid functional monomers are generally selected from ethylenically unsaturated carboxylic acids, i.e. (meth)acrylic acids. When even stronger acids are desired, acidic monomers include the ethylenically unsaturated sulfonic acids and ethylenically unsaturated phosphonic acids. Depending on the desired end use and physical properties of the final composition, the acid functional monomer may used in amounts of 5 molar equivalents or more relative to the molar equivalents of the imidazole groups. In some embodiments the molar ratio of acid groups to imidazole groups is approximately equimolar ±20%.

In some embodiments, a completely cured (i.e. hardened) polymerizable ionic liquid is solid at 25°C. In other embodiments, particularly where low $T_g$ optional monomers are used, the completely cured polymerizable ionic liquid may be a liquid at 25°C. In some embodiments the hardened composition is substantially free of uncured polymerizable ionic liquid, i.e. < 10% extractable. In preferred embodiments, the amount of uncured extractable polymerizable ionic liquid is less than 10%, more preferably less than 5%, and most preferably less than 1% by weight of the cured composition.

The polymerizable ionic liquid may also comprise other conventional (e.g. (meth)acrylate) ethylenically unsaturated monomer(s), oligomer(s), or polymer(s). By "optional monomers" is it meant an ethylenically unsaturated monomer that is not a polymerizable ionic liquid, and includes polar and nonpolar monomers and oligomers, as described more fully herein. Although conventional monomers are polymerizable and many are liquids at 25°C, conventional monomers are typically non-ionic, lacking a cation and an anion.

The total concentration of polymerizable ionic liquid(s) is typically at least 30 wt-% and preferably at least 40 wt-% of the unfilled composition (the total polymerizable organic composition excluding inorganic filler). In this embodiment, the total concentration of other ethylenically unsaturated (e.g. (meth)acrylate) monomer(s), oligomer(s), and polymer(s)) is typically at least 10 wt-%, 20 wt-%, 30 wt-%, 40 wt-%, 50 wt-%, or 65 wt-%.

The polymerizable ionic liquid composition may optionally further comprise (meth)acrylate ester monomers or oligomers. The (meth)acrylate ester monomer useful in preparing the acid functional (meth)acrylate adhesive copolymer is a monomeric (meth)acrylic ester of a non-tertiary alcohol, which alcohol contains from 1 to 14 carbon atoms and preferably an average of from 4 to 12 carbon atoms.

Examples of monomers suitable for use as the (meth)acrylate ester monomer include the esters of either acrylic acid or methacrylic acid with non-tertiary alcohols such as ethanol, 1-propanol, 2-propanol, 1-butanol, 2-butanol, 1-pentanol, 2-pentanol, 3-pentanol, 2-methyl-1-butanol, 3-methyl-1-butanol, 1-hexanol, 2-hexanol, 2-methyl-1-pentanol, 3-methyl-1-pentanol, 2-ethyl-1-butanol, 3,5,5-trimethyl-1-hexanol, 3-heptanol, 1-octanol, 2-octanol, isooctylalcohol, 2-ethyl-1-hexanol, 1-decanol, 2-propylheptanol, 1-dodecanol, 1-tridecanol, 1-tetradecanol, citronellol, dihydrocitronellol, and the like. In some embodiments, the preferred (meth)acrylate ester monomer is the ester of (meth)acrylic acid with butyl alcohol or isooctyl alcohol, or a combination thereof, although combinations of two or more different (meth)acrylate ester monomer are suitable. In some embodiments, the preferred (meth)acrylate ester monomer is the ester of (meth)acrylic acid with an alcohol derived from a renewable source, such as 2-octanol, citronellol, dihydrocitronellol.

In some embodiments it is desirable for the (meth)acrylic acid ester monomer to include a high $T_g$ monomer, having a $T_g$ of at least 25°C, and preferably at least 50°C as estimated by the Fox equation or determined by DSC. Examples of suitable monomers useful in the present invention include, but are not limited to, t-butyl acrylate, methyl methacrylate, ethyl methacrylate, isopropyl methacrylate, n-butyl methacrylate, isobutyl methacrylate, s-butyl methacrylate, t-butyl methacrylate, stearyl methacrylate, phenyl methacrylate, cyclohexyl methacrylate, isobornyl acrylate, isobornyl methacrylate, benzyl methacrylate, 3,3,5 trimethylcyclohexyl acrylate, cyclohexyl acrylate, N-octyl acrylamide, and propyl methacrylate or combinations.

The (meth)acrylate ester monomer is present in an amount of 5 to 50 parts by weight based on 100 parts total monomer content used to prepare the polymer. Preferably (meth)acrylate ester monomer is present in an amount of 5 to 40 parts by weight based on 100 parts total monomer content. When high $T_g$ monomers are included, the copolymer may include up to 30 parts by weight, preferably up to 20 parts by weight of the 5 to 50 parts by weight of (meth)acrylate ester monomer component.

The polymerizable ionic liquid may optionally further comprise polar monomers. As used herein the term "polar monomers" are exclusive of acid functional monomers.

Representative examples of suitable polar monomers include but are not limited to 2-hydroxyethyl (meth)acrylate; N-vinylpyrrolidone; N-vinylcaprolactam; acrylamide; mono- or di-N-alkyl substituted acrylamide; t-butyl acrylamide; dimethylaminoethyl acrylamide; N-octyl acrylamide; poly(alkyleneoxy) (meth)acrylates including 2-(2-ethoxyethoxy)ethyl (meth)acrylate, 2-ethoxyethyl (meth)acrylate, 2-methoxyethoxyethyl (meth)acrylate, 2-methoxyethyl methacrylate, polyethylene glycol mono(meth)acrylates; alkyl vinyl ethers, including vinyl methyl ether; and mixtures thereof. Preferred polar monomers include those selected from the group consisting of 2-hydroxyethyl (meth)acrylate and N-vinylpyrrolidinone. The polar monomer may be present in amounts of 0 to 90 parts by weight, preferably 0.5 to 50 parts by weight, based on 100 parts by weight total monomer.

The polymerizable ionic liquid may optionally further comprise vinyl monomers, and includes vinyl esters (e.g., vinyl acetate and vinyl propionate), styrene, substituted styrene (e.g., $\alpha$-methyl styrene), vinyl halide, and mixtures thereof. As used herein vinyl monomers are exclusive of acid functional monomers, acrylate ester monomers and polar monomers. Such vinyl monomers are generally used at 0 to 5 parts by weight, preferably 1 to 5 parts by weight, based on 100 parts by weight total monomer.

The polymerizable ionic liquid may optionally further comprise a multifunctional poly(meth)acryloyl monomer incorporated into the blend of polymerizable monomers as a component of the monomers. Examples of useful multifunctional (meth)acrylate include, but are not limited to, di(meth)acrylates, tri(meth)acrylates, and tetra(meth)acrylates, such as 1,6-hexanediol di(meth)acrylate, poly(ethylene glycol) di(meth)acrylates, polybutadiene di(meth)acrylate, polyurethane di(meth)acrylates, and propoxylated glycerin tri(meth)acrylate, and mixtures thereof. The amount and identity of multifunctional (meth)acrylate is tailored depending upon the particular application with those of Formula IV *supra* preferred, but where subscript v is 2-6.

Typically, the multifunctional (meth)acrylate is present in amounts less than 50 parts based on total weight of monomer composition. More specifically, the crosslinker may be present in amounts from 0.01 to 50 parts, preferably 0.05 to 20 parts, most preferably 0.05 to 5 parts, based on 100 parts total monomers of the composition.

It has been found that the amount of multifunctional (meth)acrylate crosslinking agent affects the water removability of the resulting coating. At low levels of crosslinking agent, the coating swells and breaks up into pieces. At higher levels the coating swells, but may delaminate from the substrate as a single layer.

In such embodiments, the composition may comprise:

i. 5 to 50 parts by weight of an (meth)acrylic acid ester monomers;
ii. 0.5 to 95 parts by weight of an acid functional ethylenically unsaturated monomer;
iii. 0 to 90 parts by weight of a non-acid functional, ethylenically unsaturated polar monomer;
iv. 0 to 5 parts vinyl monomer; and
v. 0 to 50 parts of a multifunctional (meth)acrylate;

based on 100 parts by weight total monomer.

Some portion of the (meth)acrylic acid ester monomer units may be hydrolyzed to acid groups after the copolymer is prepared.

In some embodiments the composition may include filler. Such compositions may include at least 40 wt-%, more preferably at least 45 wt-%, and most preferably at least 50 wt-% filler, based on the total weight of the composition. In some embodiments the total amount of filler is at most 90 wt-%, preferably at most 80 wt-%, and more preferably at most 75 wt-% filler.

In such compositions comprising appreciable amounts of filler, the one or more polymerizable ionic liquids are typically present in an amount totaling at least 5 wt-%, preferably at least 10 wt-%, based on the total weight of the composition. The concentration of multifunctional polymerizable ionic liquids is generally no greater than about 60 wt-%. In some embodiments the total amount of multifunctional polymerizable ionic liquids is at most 40 wt-%, preferably at most 30 wt-%, and more preferably at most 25 wt-%.

Fillers may be selected from one or more of a wide variety of materials, as known in the art, and include organic and inorganic filler. Inorganic filler particles include silica, submicron silica, zirconia, submicron zirconia, and non-vitreous microparticles of the type described in U.S. Pat. No. 4,503,169 (Randklev).

Filler components include nanosized silica particles, nanosized metal oxide particles, and combinations thereof. Nanofillers are also described in U.S. Pat. Nos. 7,090,721 (Craig et al.), 7,090,722 (Budd et al.), 7,156,911 (Kangas et al.), and 7,649,029 (Kolb et al.).

Fillers may be either particulate or fibrous in nature. Particulate fillers may generally be defined as having a length to width ratio, or aspect ratio, of 20:1 or less, and more commonly 10:1 or less. Fibers can be defined as having aspect ratios greater than 20:1, or more commonly greater than 100:1. The shape of the particles can vary, ranging from spherical to ellipsoidal, or more planar such as flakes or discs. The macroscopic properties can be highly dependent on the shape of the filler particles, in particular the uniformity of the shape.

In some embodiments, the composition preferably comprise a nanoscopic particulate filler (i.e., a filler that comprises nanoparticles) having an average primary particle size of less than about 0.100 micrometers (i.e., microns), and more preferably less than 0.075 microns. As used herein, the term "primary particle size" refers to the size of a non-associated single particle. The average primary particle size can be determined by cutting a thin sample of hardened composition and measuring the particle diameter of about 50-100 particles using a transmission electron micrograph at a magnification of 300,000 and calculating the average. The filler can have a unimodal or polymodal (e.g., bimodal) particle size distribution. The nanoscopic particulate material typically has an average primary particle size of at least about 2 nanometers (nm), and preferably at least about 7 nm. Preferably, the nanoscopic particulate material has an average primary particle size of no greater than about 50 nm, and more preferably no greater than about 20 nm in size. The average surface area of such a filler is preferably at least about 20 square meters per gram ($m^2/g$), more preferably, at least about 50 $m^2/g$, and most preferably, at least about 100 $m^2/g$.

In some embodiments, a combination of surface modifying agents can be useful, wherein at least one of the agents has a functional group co-polymerizable with a hardenable resin. Other surface modifying agents which do not generally react with hardenable resins can be included to enhance dispersibility or rheological properties. Examples of silanes of this type include, for example, aryl polyethers, alkyl, hydroxy alkyl, hydroxy aryl, or amino alkyl functional silanes.

Optionally, compositions may contain solvents (e.g., alcohols (e.g., propanol, ethanol), ketones (e.g., acetone, methyl ethyl ketone), esters (e.g., ethyl acetate), other nonaqueous solvents (e.g., dimethylformamide, dimethylacetamide, dimethylsulfoxide, 1-methyl-2-pyrrolidinone)), and water. When using the composition in molding operations, the composition desirably contains no solvents.

If desired, the compositions can contain additives such as indicators, dyes, pigments, inhibitors, accelerators, viscosity modifiers, wetting agents, buffering agents, radical and cationic stabilizers (for example BHT,), and other similar ingredients that will be apparent to those skilled in the art.

The polymerizable mixture comprising the ionic polymerizable liquid, acid functional monomers and "optional" monomers may be polymerized by any conventional free radical polymerization method, including solution, radiation,

bulk, dispersion, emulsion, and suspension processes. For optical applications, solution, UV and bulk processes are preferred. The resulting (co)polymers may be random or block (co)polymers.

Initiators useful in preparing the (meth)acrylate adhesive copolymers used in the present invention are initiators that, on exposure to heat, generate free-radicals which initiate (co)polymerization of the monomer mixture. Water-soluble initiators are preferred for preparing the (meth)acrylate polymers by emulsion polymerization. Suitable water-soluble initiators include but are not limited to those selected from the group consisting of potassium persulfate, ammonium persulfate, sodium persulfate, and mixtures thereof; oxidation-reduction initiators such as the reaction product of the above-mentioned persulfates and reducing agents such as those selected from the group consisting of sodium metabisulfite and sodium bisulfite; and 4,4'-azobis(4-cyanopentanoic acid) and its soluble salts (e.g., sodium, potassium). The preferred water-soluble initiator is potassium persulfate. Suitable oil-soluble initiators include but are not limited to those selected from the group consisting of azo compounds such as VAZO™ 64 (2,2'-azobis(isobutyronitrile)) and VAZO™ 52 (2,2'-azobis(2,4-dimethylpentanenitrile)), both available from E.I. du Pont de Nemours Co., peroxides such as benzoyl peroxide and lauroyl peroxide, and mixtures thereof. The preferred oil-soluble thermal initiator is (2,2'-azobis(isobutyronitrile)). When used, initiators may comprise from about 0.05 to about 1 part by weight, preferably about 0.1 to about 0.5 part by weight based on 100 parts by weight of monomer components in the pressure-sensitive adhesive.

Alternatively, the mixture can be polymerized by techniques including, but not limited to, the conventional techniques of solvent polymerization, dispersion polymerization, and solventless bulk polymerization. The monomer mixture may comprise a polymerization initiator, especially a thermal initiator or a photoinitiator of a type and in an amount effective to polymerize the comonomers, as previously described.

A typical solution polymerization method is carried out by adding the monomers, a suitable solvent, and an optional chain transfer agent to a reaction vessel, adding a free radical initiator, purging with nitrogen, and maintaining the reaction vessel at an elevated temperature, typically in the range of about 40 to 100°C until the reaction is completed, typically in about 1 to 20 hours, depending upon the batch size and temperature. Examples of the solvent are methanol, tetrahydrofuran, ethanol, isopropanol, acetone, methyl ethyl ketone, methyl acetate, ethyl acetate, toluene, xylene, and an ethylene glycol alkyl ether. Those solvents can be used alone or as mixtures thereof.

In a typical photopolymerization method, a monomer mixture may be irradiated with ultraviolet (UV) rays in the presence of a photopolymerization initiator (i.e., photoinitiators). Preferred photoinitiators are those available under the trade designations IRGACURE™ and DAROCUR™ from Ciba Speciality Chemical Corp., Tarrytown, NY and include 1-hydroxy cyclohexyl phenyl ketone (IRGACURE™ 184), 2,2-dimethoxy-1,2-diphenylethan-1-one (IRGACURE 651), *bis*(2,4,6-trimethylbenzoyl)phenylphosphineoxide (IRGACURE™ 819), 1-[4-(2-hydroxyethoxy)phenyl]-2-hydroxy-2-methyl-1-propane-1-one (IRGACURE™ 2959), 2-benzyl-2-dimethylamino-1-(4-morpholinophenyl)butanone (IRGACURE™ 369), 2-methyl-1-[4-(methylthio)phenyl]-2-morpholinopropan-1-one (IRGACURE™ 907), and 2-hydroxy-2-methyl-1-phenyl propan-1-one (DAROCUR™ 1173). Particularly preferred photoinitiators are IRGACURE™ 819, 651, 184 and 2959.

Solventless polymerization methods, such as the continuous free radical polymerization method described in U.S. Pat. Nos. 4,619,979 and 4,843,134(Kotnour et al.), the essentially adiabatic polymerization methods using a batch reactor described in U.S. Pat. No. 5,637,646 (Ellis), and, the methods described for polymerizing packaged pre-adhesive compositions described in U.S. Pat. No. 5,804,610 (Hamer et al.) may also be utilized to prepare the polymers.

As an alternative to adding the polymerizable ionic liquid to the acid-functional monomer and other optional monomers, and then polymerizing, the imidazole compound of Formula I may be added to a separately prepared, extant acid-functional copolymer comprising the acid functional and "optional" monomers described *supra*. It is believed that the amino groups of the imidazole compound of Formula I interact with the pendent acid functional groups of the acid functional (meth)acrylate copolymer to form an ionic linkage, i.e. an imidazolium group. In embodiments where the imidazole compound has two or more imidazole groups, the polymer compositions can crosslink by forming a plurality of ionic bonds between the polymer chains.

The copolymerizable mixture may optionally further comprise chain transfer agents to control the molecular weight of the resultant polymer. Examples of useful chain transfer agents include but are not limited to those selected from the group consisting of carbon tetrabromide, alcohols, mercaptans, and mixtures thereof. When present, the preferred chain transfer agents are isooctylthioglycolate and carbon tetrabromide. The polymerizable mixture may further comprise up to about 0.5 parts by weight of a chain transfer agent, typically about 0.01 to about 0.5 parts by weight, if used, preferably about 0.05 parts by weight to about 0.2 parts by weight, based upon 100 parts by weight of the total monomer mixture.

The copolymer as crosslinked by the imidazole compounds of Formula I, x=2, may be represented by:

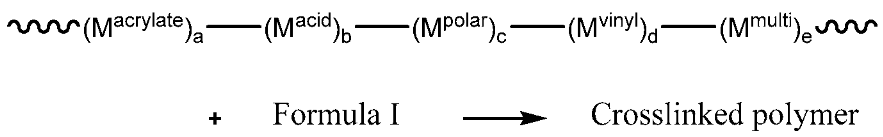


where

$M_{acrylate}$ represents polymerized (meth)acrylate monomer units derived from (meth)acrylic acid ester of non-tertiary alcohol having "a" polymerized monomer units,

Macid represents polymerized monomer units derived from acid functional monomers having "b" polymerized monomer units, shown as the conjugate base although the acid may be present;

$M_{polar}$ represents polymerized polar monomer units having "c' polymerized monomer units,

$M_{vinyl}$ represents polymerized vinyl monomer units derived from acid functional monomers having "d" polymerized monomer units, and

$M_{multi}$ represents polymerized multifunctional (meth)acrylate monomer units having "e" polymerized monomer units, and

wherein a and b are at least one and c, d, and e may be zero or non-zero, and

$R^1$ is H or a $C_1$-$C_{25}$ alkyl group,

It will be understood that the values of subscripts a to e correspond to the amounts of the monomers in the polymerizable composition, i.e. 5 to 50 parts by weight of an (meth)acrylic acid ester monomer; and 0.5 to 95 parts by weight of an acid functional monomer. Other monomers may be present in the amounts previously recited.

The compositions of the imidazole compounds of Formula I in combination with the acid-functional monomer and "other monomers" or acid-functional copolymer can be used for a variety of other uses, particularly (e.g. photo) curable coatings. A coated article can be prepared by applying the composition described herein to a substrate and curing the composition.

The coating composition can be applied to a variety of substrates. Suitable substrate materials include inorganic substrates such as glass or ceramics, natural and synthetic organic substrates such as paper, wood, as well as thermosetting or thermoplastic polymers such as polycarbonate, poly(meth)acrylate (e.g., polymethyl methacrylate or "PMMA"), polyolefins (e.g., polypropylene or "PP"), polyurethane, polyesters (e.g., polyethylene terephthalate or "PET"), polyamides, polyimides, phenolic resins, cellulose diacetate, cellulose triacetate, polystyrene, styrene-acrylonitrile copolymers, epoxies, and the like. The substrate thickness typically also will depend on the intended use. For most applications, substrate thicknesses of less than about 0.5 mm are preferred, and more preferably about 0.02 to about 0.2 mm. The substrate can be treated to improve adhesion between the substrate and curable coating compositions, e.g., chemical treatment, corona treatment such as air or nitrogen corona, plasma, flame, or actinic radiation. If desired, an optional tie layer or (e.g. polymerizable ionic liquid based) primer can be applied to the substrate to increase the interlayer adhesion.

The coating composition can be applied using a variety of conventional coating methods. Suitable coating methods include, for example, spin coating, knife coating, die coating, wire coating, flood coating, padding, spraying, roll coating, dipping, brushing, foam application, and the like. The coating is dried, typically using a forced air oven. The dried coating is at least partially and typically completely cured using an energy source.

The present disclosure provides an coating composition comprising the imidazole compound of Formula I and an acid-functional (meth)acrylate solute copolymer. In some embodiments the composition crosslinks by acid-base interactions between the acid groups of the copolymer and the nitrogen atoms of the imidazole compound.

The polymerizable ionic liquid compositions may also be used in the preparation of coatings. Some advantages include that the coatings disclosed herein (1) adhere well to a variety of optical films; (2) can be durable so as to withstand handling and manipulation as the optical device is used, e.g., to manufacture a display device; and (3) are clear and colorless, making them well suited for various light management purposes as they can be used as is or have additional agents imparted therein to provide color selection, haze, or other desired effect. Mono-imidazole compounds of Formula 1 are most useful in preparing coatings.

Generally, curable systems containing a significant amount of solvent, monomers and reactive diluents can give rise to a significant increase in density when transformed from the uncured to the cured state causing a net shrinkage in volume. As is well known, shrinkage can cause unpredictable registration in precise molding operations such as those required in dental applications. Shrinkage can also create residual stress in such articles, which can subsequently lead to stress cracking.

In some embodiments, compositions of this disclosure minimizes shrinkage and stress cracking. The low shrinkage compositions of this invention are particularly useful in molding applications or in any applications where

accurate molding and/or registration is required. In some embodiments the present disclosure provides compositions that exhibit less than 10% shrinkage, and preferably less than 8%. The compositions are low in viscosity and suitable for molding processes, including precision molding processes.

The present disclosure provides a process to transfer the pattern from a master mold to a more rigid and durable material, which then serves as a submaster for production. The method comprises: providing a master mold, preparing a patterned daughter mold from the composition of this invention; depositing a layer of a ductile metal on the patterned surface of said daughter mold; next depositing a layer of metal on the patterned surface of said daughter mold; optionally securing the article of the previous step to a support substrate; and removing the cured composition layer by an aqueous rinse to produce metal submaster mold having a patterned surface. The deposited metal may include nickel, copper, silver, gold, aluminum or their alloys.

The present invention provides methods for producing a negative or a positive submaster mold from a master. It is desirable to make submaster molds from a master mold because it enables the preparation of multiple copies of the master mold that may be used in production of shaped articles without relying on the master, and may be tiled to more efficiently produce such shaped articles.

The present invention overcomes problems in the art by providing a method that may be used repeatedly in preparing replica molds (submaster molds) from a master mold with little or no degradation in fidelity to the master. The submaster may have the same pattern of the master or the negative thereof, as desired.

It can be advantageous to apply a release coating to a mold before casting polymerizable composition to the mold. The release coating typically reduces the surface energy of the mold surface by forming a thin, thermally-stable surface that can be used to cast replication polymers and accurately reproduce microstructures and nanofeatures. In nanoreplication applications, where the pattern sizes of the mold are very small--on the order of micrometers to nanometers--conventional coating technology cannot be applied because a thick release layer on the mold can change the feature dimensions of the pattern. There are many applications for which it would be desirable to make hierarchical articles where smaller structures (nanofeatures, for example) are present upon larger structures (microstructures, for example). These applications include sensors, optical devices, fluidic devices, medical devices, molecular diagnostics, plastic electronics, micro-electromechanical systems (MEMS) and nano-electromechanical systems (NEMS). It would be advantageous to be able to mass produce microstructures, nanofeatures or hierarchical structures that contain nanofeatures and microstructures in a rapid, cost-effective, high quality manner.

In some embodiments, the mold can be coated with a fluorosilane release agent such as, for example, trimethychlorosilane or fluorinated siloxanes such as those disclosed in U. S. Pat. No. 5,851,674 (Pellerite et al.). Also useful for this purpose are hexafluoropolyprolylene oxide (HFPO) derivatives such as those disclosed in U. S. Pat. No. 7,173,778 (Jing et al.).

Alternatively, the array of mold elements can be metallized with, for example, a thin layer of metal that has been vapor deposited or deposited by electroless plating. Metals that may be deposited include nickel, copper, silver, gold, aluminum or their alloys. If the article is metallized it can also be advantageous to put a release agent on the metallized article to enhance the release of the polymers that form the replica. For example, the array of mold elements can be coated with a release layer such as a fluorinated phosphonic acid as disclosed in U.S. 6824882 (Boardman et al.) or perfluoropolyether amide-linked phosphonates such as those disclosed in U.S. No. 2005/0048288 (Flynn et al). It is also contemplated that the array of mold elements can be protected by coating with diamond- like glass as disclosed, for example in U.S. Pat. No. 6,696,157 (David et al.). Other materials that can be used as a release layer are discussed in US 20080315459 (Zhang et al.).

Such release agents may also be added to the curable composition or the coating compositions in amounts of 0.1 to 5 wt.%, relative to the weights of the compositions.

Referring now to **FIG. 1** a side view of an exemplary substrate 20 is illustrated. The substrate 20 has a first side 22 and a second side 24. While the illustrated embodiment depicts a coating only on first side 22, the casting techniques discussed below are adaptable to produce embodiments patterned on both sides if such are desired. Numerous substrates are suitable for use with the present disclosure provided they can pass the wavelengths used to cure the swellable acrylic compostions, with polyesters such as polyethylene terephthalate (PET), polyethylene naphthalate (PEN), and cyclical polyolefins (COP) being considered convenient. Substrates may be primed or unprimed.

Referring now to **FIG. 2** a side view of the substrate 20 of **FIG. 1** with a patterned coating 30 of a water-removable composition disposed on first side 22 is depicted. It will be seen that patterned coating 30 includes thick sections 32 and thin sections 34. Cast and cure techniques suitable for providing such a patterned coating are disclosed in U.S. Patents 7,165,959 (Humlicek et al); 7165959; 7224529; 7417798; and 7804649. For convenience during coating, the viscosity of the polymerizable precursor may be less than 5000 centiPoise, or even less than 1000 cP, or even less than 500 cP.

Referring now to **FIG. 3,** a side view of the substrate of **FIG. 2** is illustrated after an etching process has been performed to remove the thin portions 34 of patterned coating 30. Sufficient etching has been performed so that the thin portions 34 are completely removed all the way down to the first side 22 of substrate 20.

Etching the thin portions 34 is conveniently accomplished with reactive ion etching (RIE). Oxygen ions are

considered suitable, conveniently generated from an oxygen flow of approximately 0.5 standard liters/minute, at 200 watts of RF power from between about 20 to 60 seconds at 60 to 80 mTorr (8 to 10.66 Pascal). Fluorocarbon ion etching is also considered suitable.

Referring now to **FIG. 4,** a side view of the substrate of **FIG. 3** is illustrated after a metallization process has been performed. It will be observed that a metal layer 40 has been deposited, divided into portions 40a that are disposed direction on first side 22, and portions 40b that are disposed upon the remaining thick portions 32 of patterned coating 30.

The metallization is conveniently accomplished by sputter deposition or vapor deposition. Diverse metals may be used, including copper, silver, gold, and aluminum, and their alloys.

Referring now to **FIG. 5,** a side view of the substrate of **FIG. 4** after a water wash has been used to remove the remaining portions of the patterned coating 30. The compositions discussed above swell and often break apart into particles during the water wash, loosening them from their attachment to first surface 22. When they swell and detach, they carry away portions 40b, leaving only portions 40a in firm attachment with substrate 20 in the pattern originally defined by the thin sections 34 of the patterned coating 30. It has been found convenient to perform the water wash at a temperature of between 48.90 and 93.33 °C (120 and 200 °F).

**Examples**

**Materials Table**

| | |
|---|---|
| Imidazole | Imidazole, available from Alfa Aesar, Ward Hill, MA. |
| 2EEA | 2-ethoxyethyl acrylate, available from Sigma-Aldrich Corporation, Milwaukee, WI. |
| 2EEEA | Ethoxy-ethoxy ethyl acrylate, available under the trade designation VISCOAT 190 from Osaka Organic Chemical Industry Limited, Osaka City, Japan |
| SR 285 | Tetrahydrofurfuryl acrylate, available under the trade designation SR285 from Sartomer Americas, Exton, PA. |
| CD 553 | A liquid, monofunctional methoxylated poly(ethylene glycol) (550) acrylate monomer having a glass transition temperature of approximately -50 °C and a viscosity at 25 °C of 50 centiPoise, available under the trade designation CD 553 from Sartomer Americas, Exton, PA. |
| AA | Glacial acrylic acid, available from from BASF Corporation, Wyandotte, Michigan. |
| Acrylate Oligomer | A clear liquid acidic acrylate oligomer having a glass transition temperature of 18 °C and a viscosity at 25 °C of 115 centiPoise, available under the trade designation CN 147 from Sartomer Americas, Exton, PA. |
| 1819 | A photoinitiator, bis(2,4,6-trimethylbenzoyl)-phenylphosphineoxide, available under the trade designation IRGACURE 819 from from BASF Corporation, Wyandotte, Michigan. |
| TMPTA | A low volatility, clear liquid monomer, trimethyolpropane triacrylate, having a viscosity at 25 °C of 65 centiPoise and a glass transition temperature (DSC) of 62 °C, available under the trade designation SR 351LV from Sartomer Americas, Exton, PA. |
| TPO-L | A liquid photoinitiator, ethyl (2,4,6-trimethylbenzoyl) phenylphosphinate available under the trade designation IRGACURE TPO-L from BASF Corporation, Wyandotte, Michigan. |

Glass Transition Temperatures by Differential Scanning Calorimetry (DSC)

Open, hermetic, aluminum differential scanning calorimetry (DSC) pans containing cured (polymerized) samples of polymerizable ionic liquids (PILs), were evaluated by DSC using a TA Model DSC Q200 (available from TA Instruments, New Castle, DE) and an empty reference pan. The following temperature protocol was employed: heat from room temperature to 220 °C at a rate of 10 °C/minute, cool to -80 °C at a rate of 5 °C, heat to 220 °C at a rate of 10 °C/minute. Glass transition ($T_g$) temperatures were identified based on heat flow versus temperature during the second heat ramp. The glass transition temperature was recorded at the inflection point.

Water Removability

The ability to remove cured films, made from polymerizable ionic liquids (PILs), from a primed polyester film

backing using a brief exposure to water at room temperature was evaluated as follows. A metal punch measuring 6.1 centimeters by 1.3 centimeters was used to cut out three samples of a cured PIL film on a primed polyethylene terephthalate (polyester) backing, covered with a polyester release film liner. The release liner was removed and the remaining primed polyester backing with cured PIL film was weighed to give an initial weight. The weight of the primed polyester backing was determined from the average of eight, primed polyester film backing samples cut out in the same manner and found to be 0.0264 grams. Samples were placed in a distilled water bath at room temperature for 30 seconds or 60 seconds then removed, patted dry with lint free tissue paper, and weighed to obtain a final weight. The % weight loss was then calculated as follows:

$$\% \text{ weight loss} = (1-((\text{final wt} - 0.0264)/(\text{initial wt} - 0.0264)) \times 100$$

The average of the three samples was reported along with observations on the behavior of the cured PIL film.

**Adducts A-D: Imidazole-Michael Adducts**

<u>Preparation of Adduct A</u>

The following procedure was used to prepare of the imidazole-Michael adduct of 2-ethoxyethyl acrylate (3-imidazol-1-yl-propionic acid 2-ethoxyethyl ester).

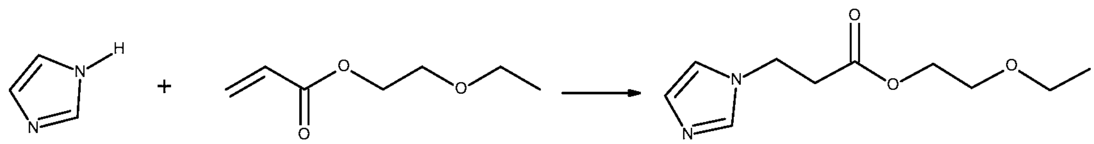

An 8 ounce (236 milliliters) glass jar was charged with 50.00 grams (0.35 moles) of 2-ethoxyethyl acrylate and 23.61 grams (0.35 moles) imidazole. The jar was heated briefly with a heat gun until the imidazole dissolved after which the jar was placed in a 70° C oven for 17 hours. NMR analysis of an aliquot of the reaction product indicated that less than 3 mole% of residual starting acrylate was present and that the reaction was complete. The reaction product was a light yellow colored, low viscosity oil. NMR analysis confirmed the structure of the product.

<u>Preparation of Adducts B-D</u>

Adducts B-D were prepared using the same general procedure as for Adduct A, and the acrylate materials shown in Table 1 below.

Table 1: Adducts

| Example | Adduct | Starting Acrylate | Imidazole-Michael Adduct |
|---|---|---|---|
| 1 | A | 2-EEA | 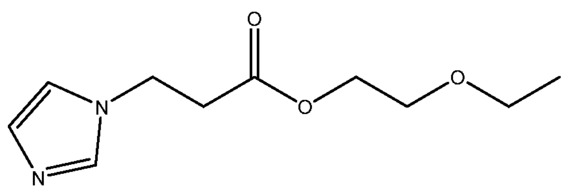 |
| 2 | B | 2-EEEA | 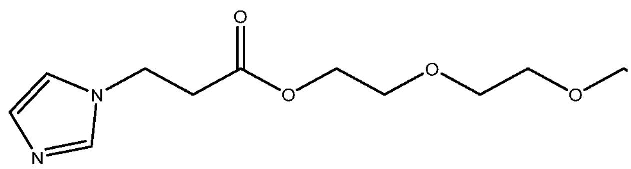 |

(continued)

| Example | Adduct | Starting Acrylate | Imidazole-Michael Adduct |
|---|---|---|---|
| 3 | C | SR 285 | 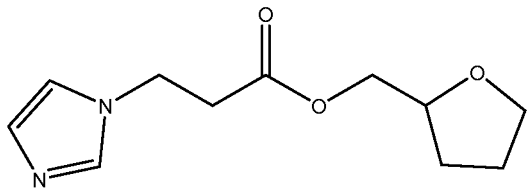 |
| 4 | D | CD 553 | 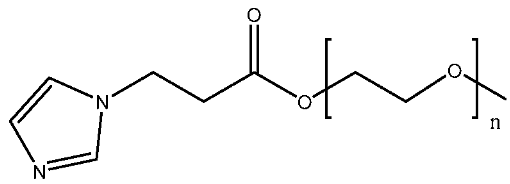 |

Examples 1-4: Polymerizable Ionic Liquids (PILs)

PIL 1 was prepared by addition of an equimolar amount of AA to Adduct A according to the following procedure. A vial was charged with 0.490 grams (2.3 millimoles) of Adduct A and 0.16 grams (2.3 millimoles) of AA. After stirring the mixture with a magnetic stir bar for about 1 hour, a clear liquid product was obtained. The product structure was confirmed by NMR.

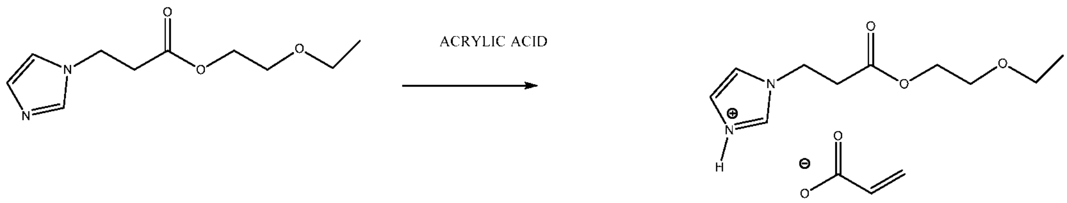


Examples 2-4 were prepared in a similar manner using the adducts shown in Table 1 above.

Each of the resulting PILs of Examples 1-4 were polymerized by mixing them with 0.2 wt% IRGACURE 819. Approximately 10 milligrams of each mixture was placed in the base of an open hermetic aluminum differential scanning calorimetry (DSC) pan (TA instruments TO91209). The pans were then placed in an enclosed chamber with a glass lid and ports through which the chamber was purged with nitrogen for 10 minutes. Next, the chamber was placed directly under a UV lamp having a peak emission at 365 nanometers and an irradiance of approximately 5 milliWatts / square centimeter for 10 minutes to fully polymerize (cure) the samples in the pans. Cured (polymerized) samples were evaluated for their glass transition temperatures by DSC as described in the test methods above. The results are shown in Table 2.

Table 2: Compositions and Thermal Properties of Examples 1-4

| Example | 1 | 2 | 3 | 4 |
|---|---|---|---|---|
| Adduct | A | B | C | D |
| Tg(°C) | 30.2 | -34.5 | 11.4 | -55.5 |
| *Example 4 also exhibited a crystallization temperature of -30 °C. | | | | |

Examples 5-8 (PILs) and Comparative Examples 1-4

Examples 5-8 and Comparative Examples 1-4 were prepared and evaluated for water removability. Various combinations of Adducts, Monomers, AA, CEA, 1819, TMPTA were thoroughly mixed for at least one hour at room temperature using a magnetic stirrer in an amber glass vial using the amounts shown in Table 3. The resulting solutions were coated onto a 25 micrometer (0.001 inch) thick, primed polyester film backing using a #14 Meyer Rod. The uncured, coated material was then covered with a silicone treated polyester release liner film. The resulting laminate construction

was then exposed to two passes at 13.7 meters/minute (45 feet/minute), from a FUSION LIGHT HAMMER 10 UV Curing System equipped with "D" bulb (Heraeus Noblelight America, Gaithersburg, MD). This corresponded to a total exposure (Joules/square centimeter) of 1.6 Joules/square centimeter UVA and 1.8 Joules/square centimeter UVV as measured by a POWER PUCK II radiometer (EIT, Incorporated, Sterling, VA). The resulting cured film laminates were evaluated as described in the Water Removability test method above.

Table 3: Compositions - Examples 5-8 and Comparative Examples 1-4

| Example | Adduct | Adduct Amount (grams) | Monomer | Monomer Amount (grams) | AA (grams) | TMPTA (grams) | I819 (grams) |
|---|---|---|---|---|---|---|---|
| CE 1 | none | 0.0 | 2EEA | 5.005 | 1.204 | 1.001 | 0.077 |
| CE 2 | none | 0.0 | 2EEEA | 4.994 | 1.410 | 1.004 | 0.075 |
| CE 3 | none | 0.0 | SR 285 | 5.018 | 1.616 | 1.001 | 0.077 |
| CE 4 | none | 0.0 | CD553 | 5.006 | 0.588 | 1.006 | 0.077 |
| 5 | A | 5.001 | none | 0.0 | 1.204 | 1.008 | 0.076 |
| 6 | B | 5.000 | none | 0.0 | 1.409 | 1.000 | 0.074 |
| 7 | C | 5.001 | none | 0.0 | 1.614 | 1.003 | 0.076 |
| 8 | D | 4.998 | none | 0.0 | 0.587 | 1.004 | 0.067 |

Table 4: Water Removability - Examples 5-8 and Comparative Examples 1-4

| Example | 30 Seconds | | 60 Seconds | |
|---|---|---|---|---|
| | weight loss (%) | Comment | weight loss (%) | Comment |
| CE 1 | -5 | Cured film swelling | -4 | Cured film swelling |
| CE 2 | -9 | Cured film swelling | -4 | Cured film swelling |
| CE 3 | -4 | Cured film swelling | -4 | Cured film swelling |
| CE 4 | 97 | Cured film broke into pieces | NT | NT |
| 5 | 77 | Cured PIL film broke into pieces | NT | NT |
| 6 | 84 | Cured PIL film broke into pieces | NT | NT |
| 7 | -124 | Cured PIL film swelling | 96 | Cured PIL film broke into pieces |
| 8 | 99 | Cured PIL film broke into pieces | NT | NT |
| NT: not tested | | | | |

Examples 9-15 (PILs)

Examples 9-15 were prepared and evaluated for water removability as described for Examples 5-8 and Comparative Examples 1-4 using the materials and amounts shown in Table 5

Table 5: Compositions - Examples 9-15

| Example | Adduct | Adduct Amount (grams) | Acrylate Oligomer (grams) | AA (grams) | TMPTA (grams) | TPO-L (grams) | I819 (grams) |
|---|---|---|---|---|---|---|---|
| 9 | B | 7.00 | 0.0 | 1.96 | 1.00 | 0.1 | 0.0 |
| 10 | B | 6.20 | 0.0 | 1.74 | 2.00 | 0.1 | 0.0 |
| 11 | B | 5.50 | 0.0 | 1.54 | 3.00 | 0.1 | 0.0 |
| 12 | B | 5.00 | 4.23 | 0.0 | 1.00 | 0.0 | 0.07 |
| 13 | D | 5.00 | 1.76 | 0.0 | 1.00 | 0.0 | 0.07 |

(continued)

| Example | Adduct | Adduct Amount (grams) | Acrylate Oligomer (grams) | AA (grams) | TMPTA (grams) | TPO-L (grams) | I819 (grams) |
|---|---|---|---|---|---|---|---|
| 14 | C | 5.00 | 4.84 | 0.0 | 1.00 | 0.0 | 0.07 |
| 15 | A | 5.00 | 3.61 | 0.0 | 1.00 | 0.0 | 0.07 |

Table 6: Water Removability - Examples 9-15

| Example | 60 Seconds | |
|---|---|---|
| | weight loss (%) | Comments |
| 9 | 83 | Cured PIL film broke into pieces |
| 10 | 87 | Cured PIL film delaminated from backing |
| 11 | 90 | Cured PIL film delaminated from backing |
| 12 | 85 | Cured PIL film broke into pieces |
| 13 | 86 | Cured PIL film broke into pieces |
| 14 | 89 | Cured PIL film broke into pieces |
| 15 | 89 | Cured PIL film broke into pieces |

Embodiments

This invention provides the following illustrative embodiments:

1. A polymerizable ionic liquid comprising a polymerizable anion and a cation of the formula:

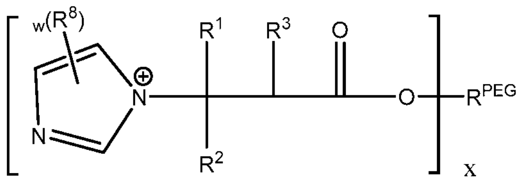

$R^1$ is H or a $C_1$-$C_{25}$ alkyl group,

$R^2$ is H or -CO- $X^1$-$R^5$, where $R^5$ is a H, a $C_1$-$C_{25}$ alkyl group or $R^{PEG}$ and $X^1$ is -O- or - $NR^6$-, where $R^6$ is H or a $C_1$-$C_6$ alkyl;

$R^3$ is H or $CH_3$,

$R^8$ is a (hetero)hydrocarbyl group, and w is 0, 1, 2 or 3; and

$R^{PEG}$ is a poly(alkyleneoxy) containing group; and

subscript x is 1 or 2.

2. The polymerizable ionic liquid of embodiment 1 wherein the polymerizable anion comprises an ethylenically unsaturated polymerizable group and an acidic group selected from a carboxylic acid group (-COOH), a sulfonic acid group ($-SO_3H$), a sulfate group ($-SO_4H$), a phosphonic acid group ($-PO_3H_2$), a phosphate group ($-OPO_3H$), or a salts thereof.

3. The polymerizable ionic liquid of any of the previous embodiments wherein said anion is a (meth)acrylate.

4. The polymerizable ionic liquid of any of the previous embodiments further comprising acid functional ethylenically unsaturated monomer.

5. The polymerizable ionic liquid any of the previous embodiments further comprising non-acid functional, ethylenically unsaturated polar monomers.

6. The polymerizable ionic liquid of any of the previous embodiments further comprising multifunctional (meth)acrylate monomers.

7. The polymerizable ionic liquid of any of the previous embodiments comprising a polymerizable mixture of:

i. 5 to 50 parts by weight of an (meth)acrylic acid ester of non-tertiary alcohol;
ii. 0.5 to 95 parts by weight of an acid functional ethylenically unsaturated monomer;
iii. 0 to 90 parts by weight of a non-acid functional, ethylenically unsaturated polar monomer;
iv. 0 to 5 parts vinyl monomer; and
v. 0 to 50 parts of a multifunctional (meth)acrylate;
based on 100 parts by weight total monomer.

8. The polymerizable ionic liquid of any of the previous embodiments wherein the molar ratio of acid groups of the acid functional ethylenically unsaturated monomer to imidazole groups is approximately equimolar ±20%.

9. The polymerizable ionic liquid of any of the previous embodiments wherein the polymerizable ionic liquid further comprises a filler.

10. The polymerizable ionic liquid of embodiment 9 wherein the filler comprises inorganic nanoparticles.

11. The polymerizable ionic liquid of any of the previous embodiments wherein said cation is prepared by the Michael addition of imidazole to a compound of the formula:

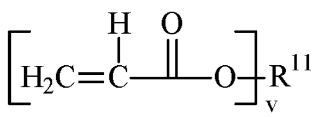
IV

$R^{11}$ is a poly(ethylene oxide) group, v is 1 or 2.

12. A method of making an article comprising:

providing the curable composition of any of any of embodiments 1-11;
applying the composition to a substrate; and
curing the composition.

13. A method of embodiment 12 comprising:

providing the curable composition of any of embodiments 1-11,
casting the curable composition in contact with a mold; and
curing the composition.

14. A coated article comprising a substrate, and a cured coating of any of the curable composition of any of any of embodiments 1-11.

15. A coating composition comprising:

a) an acid-functional (meth)acrylate copolymer, and
b) a compound of the formula:

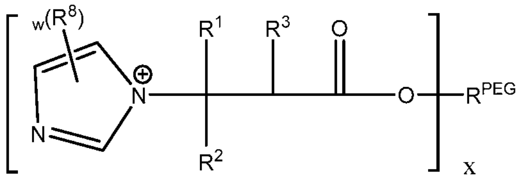

$R^1$ is H or a $C_1$-$C_{25}$ alkyl group,
$R^2$ is H or -CO- $X^1$-$R^5$, where $R^5$ is H, $C_1$-$C_{25}$ alkyl group or $R^{PEG}$; and $X^1$ is -O- or - $NR^6$-, where $R^6$ is H or a $C_1$-$C_6$ alkyl;
$R^3$ is H or $CH_3$,
$R^8$ is a (hetero)hydrocarbyl group, and w is 0, 1, 2 or 3; and
$R^{PEG}$ is a poly(alkyleneoxy) containing group;
subscript x is 1 or 2.

16. The coating composition of embodiment 15 wherein the acid-functional (meth)acrylate copolymer comprises polymerized monomer units of:

i. 5 to 50parts by weight of an (meth)acrylic acid ester monomers;
ii. 0.5 to 95 parts by weight of an acid functional ethylenically unsaturated monomers;
iii. 0 to 90 parts by weight of a non-acid functional, ethylenically unsaturated polar monomers;
iv. 0 to 5 parts vinyl monomers; and
v. 0 to 5 parts of a multifunctional (meth)acrylate monomers;
based on 100 parts by weight total monomer.

17. The coating composition of embodiment 16 wherein said non-acid functional, ethylenically unsaturated polar monomer is selected from 2-hydroxyethyl (meth)acrylate; N-vinylpyrrolidone; N-vinylcaprolactam; acrylamide; t-butyl acrylamide; dimethylamino ethyl acrylamide; N-octyl acrylamide; poly(alkyleneoxy) (meth)acrylates; poly(vinyl methyl ether); and mixtures thereof.

18. The coating composition of embodiment 16 wherein said copolymer comprises 0.5 to 95 parts by weight of acrylic acid and 1 to 50 parts by weight of a non-acid functional, ethylenically unsaturated monomer.

19. The coating composition of embodiment 18 wherein the acid functional monomer is selected from acrylic acid, methacrylic acid, itaconic acid, fumaric acid, crotonic acid, citraconic acid, maleic acid, oleic acid, β-carboxyethyl (meth)acrylate, 2-sulfoethyl methacrylate, styrene sulfonic acid, 2-acrylamido-2-methylpropane sulfonic acid, vinyl phosphonic acid, and mixtures thereof.

20. The coating composition of embodiment 16 comprising 1 to 5 parts of a vinyl monomer selected from vinyl esters, styrene, substituted styrene, vinyl halide, vinyl propionate, and mixtures thereof.

21. The coating composition of embodiment 16 wherein said non-tertiary alcohol of said (meth)acrylic acid ester of non-tertiary alcohol is selected from 2-octanol or dihydrocitronellol.

22. The coating composition of any of embodiments 16-21 wherein the acid-functional (meth)acrylate copolymer is of the formula:

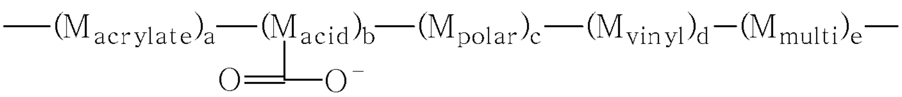

where

$M_{acrylate}$ represents polymerized multifunctional (meth)acrylate monomer units derived from (meth)acrylic acid ester of non-tertiary alcohol having "a" polymerized monomer units,
$M_{acid}$ represents polymerized monomer units derived from acid functional monomers having "b" polymerized monomer units,
$M_{polar}$ represents polymerized polar monomer units having "c' polymerized monomer units,

$M_{vinyl}$ represents polymerized vinyl monomer units derived from acid functional monomers having "d" polymerized monomer units, and
$M_{multi}$ represents polymerized multifunctional (meth)acrylate monomer units having "e" polymerized monomer units, and
wherein a and b are at least one and c, d, and e may be zero or non-zero.

23. A method of replicating a mold comprising the steps of:

a) providing a master mold;
b) depositing a layer of the curable composition of any of embodiments 1-11 and curing;
c) removing the cured patterned daughter mold;
d) optionally etching the cured composition;
e) optionally depositing a layer of a ductile metal on the patterned surface of said daughter mold;
f) depositing a metal layer of nickel, copper, silver, gold, aluminum or their alloys on the patterned surface of said daughter mold;
g) optionally securing the article of step c to a substrate;
h) removing the cured patterned daughter mold from the patterned metal mold by contacting with water.

24. The method of embodiment 23 wherein said ductile metal layer is a vapor deposited metal layer having a thickness of greater than 10 and less than 100 nanometers and a mean surface roughness of less than 10.

25. The method of embodiment 23 where in the thickness of the deposited metal layer is 0.2 mm to 5 mm.

26. The method of embodiment 23 wherein the step of depositing a layer of a ductile metal is a vapor depositing step.

27. The method of replicating a mold comprising the steps of:

a) providing a master mold have a positive pattern on the surface thereof;
b) contacting the master mold with the curable composition of any of embodiments 1- and curing,
c) removing the cured composition having a negative pattern on the surface thereof,
d) first depositing a layer of a ductile metal on the negative patterned surface;
e) next depositing a metal layer of nickel, copper, silver, gold, aluminum or their alloys on the negative patterned surface having a ductile metal layer;
f) optionally securing the article of step e) to a support substrate;
g) separating the deposited metal layer having a positive patterned surface from said cured composition to produce a first generation daughter mold,
said patterned surface having a layer of a ductile metal thereon.

## Claims

**1.** A water removable coating composition comprising a polymerizable ionic liquid comprising a polymerizable anion and a cation of the formula:

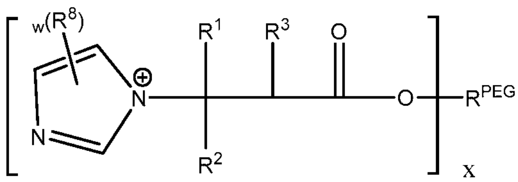

$R^1$ is H or a $C_1$-$C_{25}$ alkyl group,
$R^2$ is H or -CO- $X^1$-$R^5$, where $R^5$ is H, a $C_1$-$C_{25}$ alkyl group or $R^{PEG}$; and $X^1$ is -O- or -$NR^6$-, where $R^6$ is H or a $C_1$-$C_6$ alkyl;
$R^3$ is H or $CH_3$,
$R^8$ is a (hetero)hydrocarbyl group, and w is 0, 1, 2 or 3; and
$R^{PEG}$ is a poly(alkyleneoxy) containing group;
subscript x is 1 or 2.

**2.** The water removable coating composition of claim 1 further comprising acid functional ethylenically unsaturated monomer.

**3.** The water removable coating composition of claim 1 further comprising non-acid functional, ethylenically unsaturated polar monomers.

**4.** The water removable coating composition of claim 1 further comprising multifunctional (meth)acrylate monomers.

**5.** The water removable coating composition of claim 1 comprising a polymerizable mixture of:

i. 5 to 50 parts by weight of an (meth)acrylic acid ester of non-tertiary alcohol;
ii. 0.5 to 95 parts by weight of an acid functional ethylenically unsaturated monomer;
iii. 0 to 90 parts by weight of a non-acid functional, ethylenically unsaturated polar monomer;
iv. 0 to 5 parts vinyl monomer; and
v. 0 to 50 parts of a multifunctional (meth)acrylate;
based on 100 parts by weight total monomer.

**6.** The water removable coating composition of claim 1 wherein the molar ratio of acid groups of the acid functional ethylenically unsaturated monomer to imidazole groups is approximately equimolar ±20%.

**7.** The water removable coating composition of claim 1 wherein said cation is prepared by the Michael addition of imidazole to a compound of the formula:

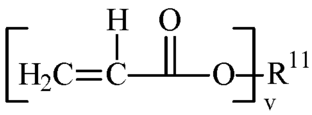

$R^{11}$ is a poly(ethylene oxide) group, v is 1 or 2.

**8.** A coated article comprising a substrate, and a cured coating of any the water removable coating composition of any of claims 1-7.

**9.** A water removable coating composition comprising:

a) an acid-functional (meth)acrylate copolymer, and
b) a compound of the formula:

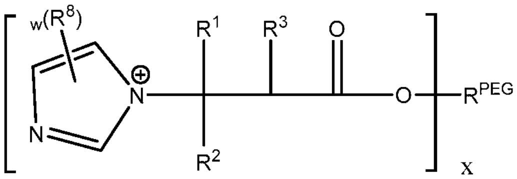

$R^1$ is H or a $C_1$-$C_{25}$ alkyl group,
$R^2$ is H or -CO- $X^1$-$R^5$, where $R^5$ is H, a $C_1$-$C_{25}$ alkyl group or $R^{PEG}$; and $X^1$ is -O- or -$NR^6$-, where $R^6$ is H or a $C_1$-$C_6$ alkyl;
$R^3$ is H or $CH_3$,
$R^8$ is a (hetero)hydrocarbyl group, and w is 0, 1, 2 or 3; and
$R^{PEG}$ is a poly(alkyleneoxy) containing group;
subscript x is 1 or 2.

**10.** The coating composition of claim 9 wherein the acid-functional (meth)acrylate copolymer comprises polymerized monomer units of:

a) 5 to 50 parts by weight of an (meth)acrylic acid ester monomers;
b) 0.5 to 95 parts by weight of an acid functional ethylenically unsaturated monomers;

c) 0 to 90 parts by weight of a non-acid functional, ethylenically unsaturated polar monomers;
d) 0 to 5 parts vinyl monomers; and
e) 0 to 5 parts of a multifunctional (meth)acrylate monomers;
based on 100 parts by weight total monomer.

**11.** The coating composition of claim 10 wherein said copolymer comprises 0.5 to 95 parts by weight of acrylic acid and 1 to 50 parts by weight of a non-acid functional, ethylenically unsaturated monomer.

**12.** The coating composition of claim 10 wherein the acid-functional (meth)acrylate copolymer is of the formula:

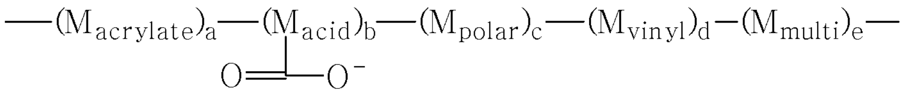

where

$M_{acrylate}$ represents polymerized multifunctional (meth)acrylate monomer units derived from (meth)acrylic acid ester of non-tertiary alcohol having "a" polymerized monomer units,
$M_{acid}$ represents polymerized monomer units derived from acid functional monomers having "b" polymerized monomer units,
$M_{polar}$ represents polymerized polar monomer units having "c' polymerized monomer units,
$M_{vinyl}$ represents polymerized vinyl monomer units derived from acid functional monomers having "d" polymerized monomer units, and
$M_{multi}$ represents polymerized multifunctional (meth)acrylate monomer units having "e" polymerized monomer units, and
wherein a and b are at least one and c, d, and e may be zero or non-zerO

**13.** A method of replicating a mold comprising the steps of:

a) providing a master mold;
b) depositing a layer of the curable composition of any of claims 1-7;
c) curing
d) removing the cured patterned daughter mold;
e) optionally etching the cured composition;
f) optionally depositing a layer of a ductile metal on the patterned surface of said daughter mold;
g) depositing one or more metal layers of nickel, copper, silver, gold, aluminum or their alloys on the patterned surface of said daughter mold;
h) optionally securing the article of step c to a substrate;
i) removing the cured patterned daughter mold from the patterned mold by contacting with water.

**14.** The method of replicating a mold comprising the steps of:

i. providing a master mold have a positive pattern on the surface thereof;
ii. contacting the master mold with the curable composition of any of claims 1-7 and curing,
iii. removing the cured composition having a negative pattern on the surface thereof,
iv. optionally etching the cured composition;
v. optionally first depositing a layer of a ductile metal on the negative patterned surface;
vi. next depositing one or more metal layers of nickel, copper, silver, gold, aluminum or their alloys on the negative patterned surface having an optional ductile metal layer;
vii. optionally securing the article of step e) to a support substrate;
viii. separating the deposited metal layer having a positive patterned surface from said cured composition to produce a first generation daughter mold.

**15.** A method of replicating a mold comprising the steps of:

a) providing a master mold;
b) depositing a layer of the curable composition of any of claims 1-7;
c) removing the cured patterned daughter mold;

d) optionally depositing a layer of a ductile metal on the patterned surface of said daughter mold;
e) optionally etching the cured composition;
f) depositing one or more layers of a dielectric oxide on the patterned surface of said daughter mold;
g) optionally securing the article of step c to a substrate;
h) removing the cured patterned daughter mold from the patterned nickel mold by contacting with water.

## Patentansprüche

1. Wasserentfernbare Beschichtungszusammensetzung, umfassend eine polymerisierbare ionische Flüssigkeit, umfassend ein polymerisierbares Anion und ein Kation der Formel:

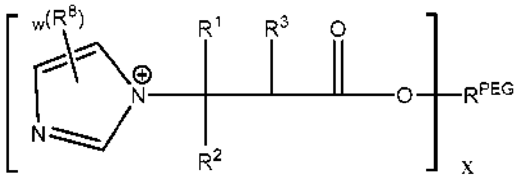

$R^1$ ist H oder eine $C_1$-$C_{25}$-Alkylgruppe,
$R^2$ ist H oder -CO- $X^1$-$R^5$, worin $R^5$ H, eine $C_1$-$C_{25}$-Alkylgruppe oder $R^{PEG}$ ist; und $X^1$ ist -O- oder -$NR^6$-, worin $R^6$ H oder ein $C_1$-$C_6$-Alkyl ist;
$R^3$ ist H oder $CH_3$,
$R^8$ ist eine (Hetero)hydrocarbylgruppe, und w ist 0, 1, 2 oder 3; und
$R^{PEG}$ ist eine Poly(alkylenoxy)-enthaltende Gruppe;
das tiefgestellte x ist 1 oder 2.

2. Wasserentfern bare Beschichtungszusammensetzung nach Anspruch 1, ferner umfassend ein säurefunktionelles ethylenisch ungesättigtes Monomer.

3. Wasserentfernbare Beschichtungszusammensetzung nach Anspruch 1, ferner umfassend nicht säurefunktionelle ethylenisch ungesättigte polare Monomere.

4. Wasserentfern bare Beschichtungszusammensetzung nach Anspruch 1, ferner umfassend multifunktionelle (Meth)acrylatmonomere.

5. Wasserentfern bare Beschichtungszusammensetzung nach Anspruch 1, umfassend ein polymerisierbares Gemisch von:

i. zu 5 bis 50 Gewichtsteilen ein (Meth)acrylsäureester von nichttertiärem Alkohol;
ii. zu 0,5 bis 95 Gewichtsteilen ein säurefunktionelles, ethylenisch ungesättigtes Monomer;
iii. zu 0 bis 90 Gewichtsteilen ein nicht säurefunktionelles, ethylenisch ungesättigtes, polares Monomer;
iv. zu 0 bis 5 Teilen Vinylmonomer; und
v. zu 0 bis 50 Teilen ein multifunktionelles (Meth)acrylat;
basierend auf 100 Gewichtsteilen Gesamtmonomer.

6. Wasserentfern bare Beschichtungszusammensetzung nach Anspruch 1, wobei das Molverhältnis von Säuregruppen des säurefunktionellen ethylenisch ungesättigten Monomers zu Imidazolgruppen etwa äquimolar±20 % beträgt.

7. Wasserentfern bare Beschichtungszusammensetzung nach Anspruch 1, wobei das Kation durch Michael-Addition von Imidazol an eine Verbindung der Formel hergestellt wird:

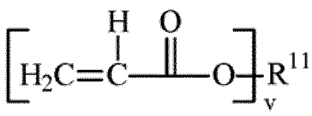

$R^{11}$ ist eine Poly(ethylenoxid)-Gruppe, v ist 1 oder 2.

**8.** BeschichteterGegenstand, umfassend ein Substrat und eine gehärtete Beschichtung aus einer beliebigen der wasserentfernbaren Beschichtungszusammensetzung nach einem der Ansprüche 1 bis 7.

**9.** Wasserentfern bare Beschichtungszusammensetzung, umfassend:

a) ein säurefunktionelles (Meth)acrylat-Copolymer und
b) eine Verbindung der Formel:

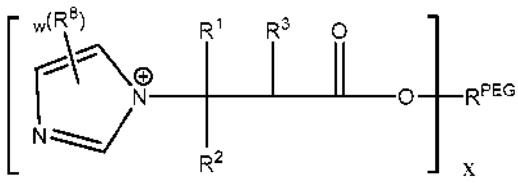

$R^1$ ist H oder eine $C_1$-$C_{25}$-Alkylgruppe,
$R^2$ ist H oder -CO- $X^1$-$R^5$, worin $R^5$ H, eine $C_1$-$C_{25}$-Alkylgruppe oder $R^{PEG}$ ist; und $X^1$ ist -O- oder -$NR^6$-, worin $R^6$ H oder ein $C_1$-$C_6$-Alkyl ist;
$R^3$ ist H oder $CH_3$,
$R^8$ ist eine (Hetero)hydrocarbylgruppe, und w ist 0, 1, 2 oder 3; und
$R^{PEG}$ ist eine Poly(alkylenoxy)-enthaltende Gruppe;
das tiefgestellte x ist 1 oder 2.

**10.** Beschichtungszusammensetzung nach Anspruch 9, wobei das säurefunktionelle (Meth)acrylat-Copolymer polymerisierte Monomereinheiten umfasst von:

a) zu 5 bis 50 Gewichtsteilen ein (Meth)acrylsäureester-Monomer;
b) zu 0,5 bis 95 Gewichtsteilen ein säurefunktionelles, ethylenisch ungesättigtes Monomer;
c) zu 0 bis 90 Gewichtsteilen ein nichtsäurefunktionelles, ethylenisch ungesättigtes, polares Monomer;
d) zu 0 bis 5 Teilen Vinylmonomere; und
e) zu 0 bis 5 Teilen ein multifunktionelles (Meth)acrylat-Monomer;
basierend auf 100 Gewichtsteilen Gesamtmonomer.

**11.** Beschichtungszusammensetzung nach Anspruch 10, wobei das Copolymer zu 0,5 bis 95 Gewichtsteilen Acrylsäure und zu 1 bis 50 Gewichtsteilen ein nicht säurefunktionelles, ethylenisch ungesättigtes Monomer umfasst.

**12.** Beschichtungszusammensetzung nach Anspruch 10, wobei das säurefunktionelle (Meth)acrylat-Copolymerder Formel entspricht:

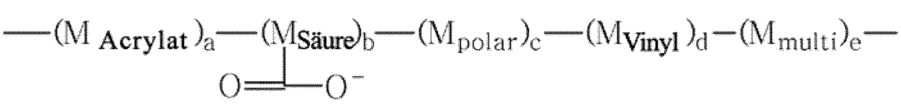

worin

$M_{Acrylat}$ für polymerisierte multifunktionelle (Meth)acrylat-Monomereinheiten steht, die von (Meth)acrylsäureester von nicht tertiärem Alkohol mit "a"-polymerisierten Monomereinheiten abgeleitetsind,
$M_{Säure}$ fürpolymerisierte Monomereinheiten steht, die von säurefunktionellen Monomeren mit "b"-polymerisierten Monomereinheiten abgeleitet sind,
$M_{polar}$ für polymerisierte polare Monomereinheiten mit "c"-polymerisierten Monomereinheiten steht,
$M_{Vinyl}$ fürpolymerisierte Vinyl-Monomereinheiten steht, die von säurefunktionellen Monomeren mit "d"-polymerisierten Monomereineiten abgeleitet sind, und
$M_{multi}$ fürpolymerisierte multifunktionelle (Meth)acrylat-Monomereinheiten mit "e"-polymerisierten Monomereinheiten steht, und
worin a und b mindestens Eins sind und c, d und e Null oder nicht Null sein können

**13.** Verfahren zum Replizieren einer Form, umfassend die Schritte:

a) Bereitstellen einer Mutterform;
b) Abscheiden einerSchichtder härtbaren Zusammensetzung nach einem der Ansprüche 1 bis 7;
c) Aushärten
d) Entfernen der gehärteten strukturierten Tochterform;
e) wahlweise Ätzen der gehärteten Zusammensetzung;
f) wahlweise Abscheiden einer Schicht eines verformbaren Metalls auf die strukturierte Oberfläche der Tochterform;
g) Abscheiden einer oder mehrerer Metallschichten aus Nickel, Kupfer, Silber, Gold, Aluminium oderderen Legierungen auf die strukturierte Oberfläche der Tochterform;
h) wahlweise Befestigen des Gegenstandes aus Schritte an einem Substrat;
i) Entfernen der gehärteten strukturierten Tochterform ausder strukturierten Form durch Inkontaktbringen mit-Wasser.

**14.** Verfahren zum Replizieren einer Form, umfassenddie Schritte:

i. Bereitstellen einer Mutterform, die ein positives Muster auf ihrer Oberfläche aufweist;
ii. Inkontaktbringen der Mutterform mit der härtbaren Zusammensetzung nach einem der Ansprüche 1 bis 7 und Aushärten,
iii. Entfernen der gehärteten Zusammensetzung, die ein negatives Muster auf ihrer Oberfläche aufweist,
iv. wahlweise Ätzen der gehärteten Zusammensetzung;
v. wahlweise zuerst Abscheiden einer Schicht eines verformbaren Metalls auf der negativen strukturierten Oberfläche;
vi. als Nächstes Abscheiden einer oder mehrerer Metallschichten aus Nickel, Kupfer, Silber, Gold, Aluminium oder ihren Legierungen auf der negativen strukturierten Oberfläche, die eine wahlweise verformbare Metallschicht aufweist;
vii. wahlweise Befestigen des Gegenstandes aus Schritte) an einem Trägersubstrat;
viii. Abtrennen der abgeschiedenen Metallschicht, die eine positiv strukturierte Oberfläche aufweist, von der gehärteten Zusammensetzung, um eine Tochterform der ersten Generation herzustellen.

**15.** Verfahren zum Replizieren einer Form, umfassend die Schritte:

a) Bereitstellen einer Mutterform;
b) Abscheiden einerSchichtder härtbaren Zusammensetzung nach einem der Ansprüche 1 bis 7;
c) Entfernen dergehärteten strukturierten Tochterform;
d) wahlweise Abscheiden einer Schichteines verformbaren Metalls auf die strukturierte Oberfläche der Tochterform;
e) wahlweise Ätzen dergehärteten Zusammensetzung;
f) wahlweise Abscheiden einer oder mehrerer Schichten eines dielektrischen Oxids auf die strukturierte Oberfläche der Tochterform;
g) wahlweise Befestigen des Gegenstandes aus Schritte an einem Substrat;
h) Entfernen dergehärteten strukturierten Tochterform von der strukturierten Nickelform durch In kontaktbringen mit Wasser.

## Revendications

**1.** Composition de revêtement pouvant être retirée à l’eau comprenant un liquide ionique polymérisable comprenant un anion polymérisable et un cation de formule :

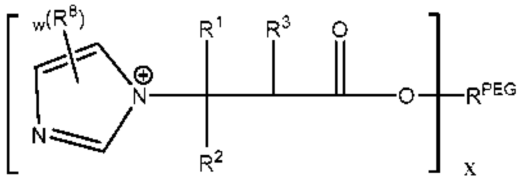

$R^1$ est H ou un groupe alkyle en $C_1$ à $C_{25}$,
$R^2$ est H ou -CO- $X^1$-$R^5$, où $R^5$ est H, un groupe alkyle en $C_1$ à $C_{25}$ ou $R^{PEG}$ ; et $X^1$ est -O- ou -$NR_6$-, où $R^6$ est

H ou un alkyle en $C_1$ à $C_6$ ;
$R^3$ est H ou $CH_3$,
$R^8$ est un groupe (hétéro)hydrocarbyle, et w vaut 0, 1, 2 ou 3 ; et
$R^{PEG}$ est un groupe contenantun poly(alkylène-oxy) ;
l'indice x vaut 1 ou 2.

**2.** Composition de revêtement pouvant être retirée à l'eau selon la revendication 1 comprenant en outre un monomère à insaturation éthylénique à fonction acide.

**3.** Composition de revêtement pouvant être retirée à l'eau selon la revendication 1 comprenant en outre des monomères polaires à insaturation éthylénique sans fonction acide.

**4.** Composition de revêtement pouvant être retirée à l'eau selon la revendication 1 comprenant en outre des monomères (méth)acrylate multifonctionnels.

**5.** Composition de revêtement pouvant être retirée à l'eau selon la revendication 1 comprenant un mélange polymérisable de :

i. 5 à 50 parties en poids d'un ester d'acide (méth)acrylique d'alcool non tertiaire ;
ii. 0,5 à 95 parties en poids d'un monomère à insaturation éthylénique à fonction acide ;
iii. 0 à 90 parties en poids d'un monomère polaire à insaturation éthylénique à fonction non acide ;
iv. 0 à 5 parties d'un monomère vinylique ; et
v. 0 à 50 parties d'un (méth)acrylate multifonctionnel ;
sur la base de 100 parties en poids de monomère total.

**6.** Composition de revêtement pouvant être retirée à l'eau selon la revendication 1 dans laquelle le rapport molaire des groupes acides du monomère à insaturation éthylénique à fonction acide aux groupes imidazole est approximativement équimolaire ±20 %.

**7.** Composition de revêtement pouvant être retirée à l'eau selon la revendication 1 dans laquelle ledit cation est préparé par l'addition de Michael d'imidazole à un composé de formule :

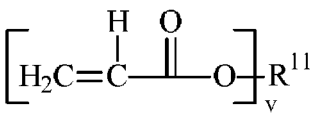

$R^{11}$ est un groupe poly(oxyde d'éthylène), v vaut 1 ou 2.

**8.** Article revêtu comprenant un substrat, et un revêtement durci de l'une quelconque composition de revêtement pouvant être retirée à l'eau selon l'une quelconque des revendications 1 à 7.

**9.** Composition de revêtement pouvant être retirée à l'eau comprenant :

a) un copolymère de (méth)acrylate à fonction acide, et
b) un composé de formule :

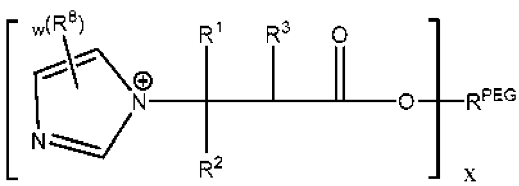

$R^1$ est H ou un groupe alkyle en $C_1$ à $C_{25}$,
$R^2$ est H ou -CO- $X^1$-$R^5$, où $R^5$ est H, un groupe alkyle en $C_1$ à $C_{25}$ ou $R^{PEG}$ ; et $X^1$ est -O- ou -$NR^6$-, où $R^6$ est H ou un alkyle en $C_1$ à $C_6$ ;
$R^3$ est H ou $CH_3$,

$R^8$ est un groupe (hétéro)hydrocarbyle, et w vaut 0, 1, 2 ou 3 ; et
$R^{PEG}$ est un groupe contenantun poly(alkylène-oxy) ;
l'indice x vaut 1 ou 2.

**10.** Composition de revêtement selon la revendication 9, dans laquelle le copolymère de (méth)acrylate à fonction acide comprend des motifs monomères polymérisés de :

a) 5 à 50 parties en poidsde monomères d'un ester d'acide (méth)acrylique ;
b) 0,5 à 95 parties en poids de monomères à insaturation éthylénique à fonction acide ;
c) 0 à 90 parties en poids de monomères polaires à insaturation éthylénique sans fonction acide ;
d) 0 à 5 parties de monomères vinyliques ; et
e) 0 à 5 parties de monomères (méth)acrylate multifonctionnels ;
sur la base de 100 parties en poids de monomère total.

**11.** Composition de revêtement selon la revendication 10 dans laquelle ledit copolymère comprend 0,5 à 95 parties en poids d'acide acrylique et 1 à 50 parties en poids d'un monomère à insaturation éthylénique sans fonction acide.

**12.** Composition de revêtement selon la revendication 10, dans laquelle le copolymère de (méth)acrylate à fonction acide est de formule :

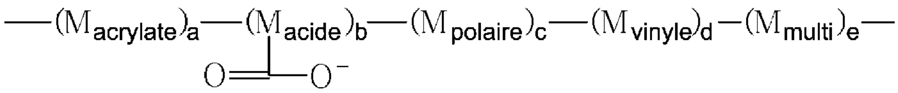

où

$M_{acrylate}$ représente des motifs monomères de (méth)acrylate multifonctionnel polymérisés, dérivés d'un ester d'acide (méth)acrylique d'alcool non tertiaire comportant des motifs monomères polymérisés « a »,
$M_{acide}$ représente des motifs monomères polymérisés dérivés de monomères à fonction acide comportant des motifs monomères polymérisés « b »,
$M_{polaire}$ représente des motifs monomères polaires polymérisés comportant des motifs monomères polymérisés « c »,
$M_{vinyle}$ représente des motifs mon omères vinyliques polymérisés dérivés de monomères à fonction acide comportant des motifs monomères polymérisés « d », et
$M_{multi}$ représente des motifs monomères de (méth)acrylate multifonctionnel polymérisés comportant des motifs monomères polymérisés « e », et
dans laquelle a et b valent au moins un et c, d et e peuvent être zéro ou différents de zéro.

**13.** Procédé de reproduction d'un moule comprenant les étapes consistant à :

a) fournir un moule maître ;
b) déposer une couche de la composition durcissable selon l'une quelconque des revendications 1 à 7 ;
c) effectuer un durcissement
d) retirer le moule enfantà motif durci ;
e) éventuellement attaquer chimiquement la composition durcie ;
f) éventuellement déposer une couche d'un métal ductile sur la surface à motif dudit moule enfant ;
g) déposer une ou plusieurs couches métalliques de nickel, de cuivre, d'argent, d'or, d'aluminium ou de leurs alliages sur la surface à motif dudit moule enfant;
h) éventuellement fixer l'article de l'étape c à un substrat;
i) retirer le moule enfantà motif durci du moule à motif par mise en contact avec de l'eau.

**14.** Procédé de reproduction d'un moule comprenant les étapes consistantà :

i. fournir un moule maître ayant un motif positif sur la surface de celui-ci ;
ii. mettre en contact le moule maître avec la composition durcissable selon l'une quelconque des revendications 1 à 7 et effectuer un durcissement,
iii. retirer la composition durcie ayant un motif négatif sur la surface de celui-ci,
iv. éventuellement attaquer chimiquement la composition durcie ;

v. éventuellement déposer d'abord une couche d'un métal ductile sur la surface à motif négatif ;
vi. ensuite déposer une ou plusieurs couches métalliques de nickel, de cuivre, d'argent, d'or, d'aluminium ou de leurs alliages sur la surface à motif négatif ayant une couche de métal ductile éventuelle ;
vii. éventuellement fixer l'article de l'étape e) à un substrat de support ;
viii. séparer la couche métallique déposée ayant une surface à motif positif par rapport à ladite composition durcie pour produire un moule enfantde première génération.

**15.** Procédé de reproduction d'un moule comprenant les étapes consistant à :

a) fournirun moule maître ;
b) déposer une couche de la composition durcissable selon l'une quelconque des revendications 1 à 7 ;
c) retirer le moule enfantà motif durci ;
d) éventuellement déposer une couche d'un métal ductile sur la surface à motif dudit moule enfant ;
e) éventuellement attaquer chimiquement la composition durcie ;
f) déposer une ou plusieurs couches d'un oxyde diélectrique sur la surface à motif dudit moule enfant ;
g) éventuellement fixer l'article de l'étape c à un substrat;
h) retirer le moule enfantà motif durci du moule en nickel à motif par mise en contact avec de l'eau.

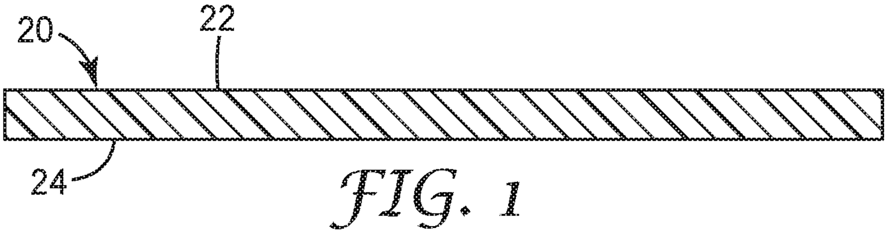


FIG. 1

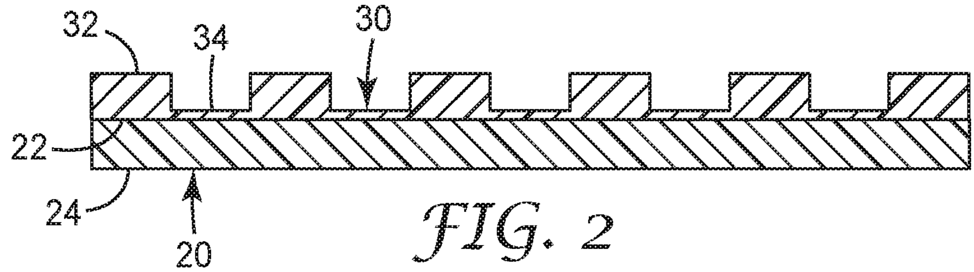


FIG. 2

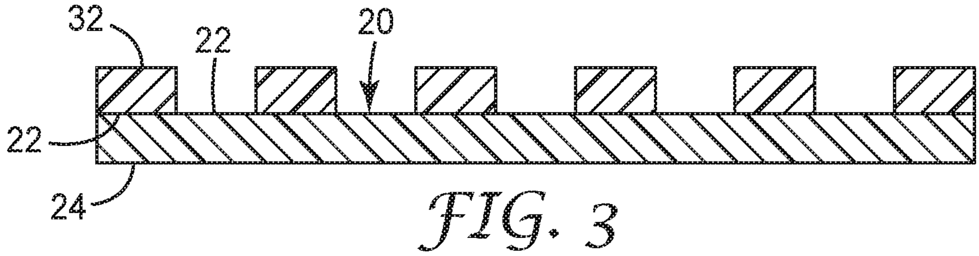


FIG. 3

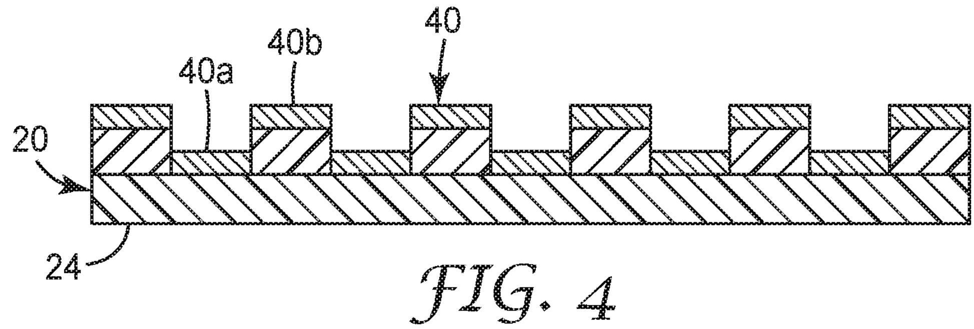


FIG. 4

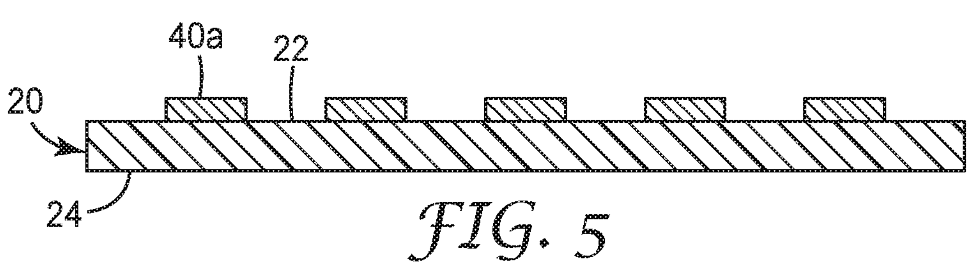


FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2011288227 A1
- US 2009029162 A1
- US 2012101184 A1
- US 4503169 A, Randklev
- US 7090721 B, Craig
- US 7090722 B, Budd
- US 7156911 B, Kangas
- US 7649029 B, Kolb
- US 4619979 A
- US 4843134 A, Kotnour
- US 5637646 A, Ellis
- US 5804610 A, Hamer
- US 5851674 A, Pellerite
- US 7173778 B, Jing
- US 6824882 B, Boardman
- US 20050048288 A, Flynn
- US 6696157 B, David
- US 20080315459 A, Zhang
- US 7165959 B, Humlicek
- US 7224529 B
- US 7417798 B
- US 7804649 B